# EUROPEAN PATENT APPLICATION

(11) **EP 3 766 432 A1**
(43) Date of publication of application: **20.01.2021**
(21) Application number: 20186064.0
(22) Date of filing: 15.07.2020
(51) Int. Cl.: A61B 17/068, H05K 3/28, H05K 1/18, A61B 17/115

(54) **SENSOR ENCAPSULATING ASSEMBLY**

(30) Priority: 16.07.2019 US 201962874550 P; 20.05.2020 US 202016879343
(71) Applicant: Covidien LP, Mansfield, MA 02048 (US)
(72) Inventor: SGROI, Anthony, Wallingford, Connecticut 06492 (US); VALENTINE, David, Hamden, Connecticut 06518 (US)
(74) Representative: Maschio, Antonio

(57) **Abstract**

A system includes a wiring harness and an encapsulation assembly for encapsulating a connection region between electronic components of the wiring harness. The wiring harness includes a force sensor electrically coupled to a flex cable at a connection region. The encapsulation assembly includes a mold having a first and second mold halves configured to retain the force sensor therein and define a cavity above the connection region of the wiring harness.

## Description

### CROSS-REFERENCE TO RELATED APPLICATIONS

This application claims the benefit of and priority to U.S. Provisional Patent Application Serial No. 62/874,550 filed July 16, 2019, the entire disclosure of which is incorporated by reference herein.

### TECHNICAL FIELD

The present disclosure relates generally to reusable surgical devices. More particularly, the present disclosure relates to powered surgical devices with enhanced durability and increased moisture resistance.

### BACKGROUND

Powered surgical devices include electronic components, such as printed circuit boards, switches, sensors, etc., to enhance the control of functions of the surgical devices. The intelligence of such surgical devices result in a higher product cost compared to currently available disposable units. Accordingly, it would be beneficial if such intelligent devices are reusable.

For example, surgical devices in the form of surgical stapling apparatus are employed by surgeons to sequentially or simultaneously apply one or more rows of fasteners, e.g., staples or two-part fasteners, to body tissue for the purpose of joining segments of body tissue together and/or attaching a surgical implant to body tissue. Disposable surgical stapling apparatus are used to perform various stapling functions in surgery, such as performing anastomosis of tubular body structures (e.g., the colon, the stomach, the small intestine, etc.) in an end to end, end to side, or side to side manner. Once the procedure is complete, the device is discarded. Reusable surgical stapling apparatus are also used to perform various stapling functions in surgery, and the use thereof may result in lower cost of a procedure over the life of the device.

Powered surgical stapling apparatus include electronic components to monitor and facilitate functions, such as clamping, stapling, and/or cutting forces of the device. For example, load reading sensors can be used to detect pre-set loads and cause the device to react to such a response. For instance, during clamping of thick tissue, the load will rise to a pre-determined limit where the device can slow clamping to maintain the clamping force as the tissue relaxes. This allows for clamping of thick tissue without damage to such tissue (e.g., serosa tears).

Reusable surgical devices must be cleaned and sterilized prior to subsequent uses. Cleaning and sterilization procedures, however, are aggressive in nature. Cleaning (e.g., washing and/or disinfecting) utilizes alkaline solutions having high pH values (e.g., a pH of 11). Autoclaving (a common method of sterilization) utilizes high pressure superheated steam (e.g., 30 PSI @ 160°C for 20 minutes). Such environments are known to damage various electronic components. For example, surgical devices may suffer from moisture ingress during cleaning and/or sterilizing procedures which, in turn, may corrode and/or degrade the electronic components.

The electronic components of reusable surgical devices may be protected from high temperatures, steam, and/or moisture by utilizing, for example, conformal coatings or potting compounds. The electronic components, however, may still suffer from moisture ingress during cleaning and/or sterilizing procedures (e.g., cracking or delamination of conformal coatings), and/or may be damaged during application of the protective materials (e.g., heat damage).

Thus, it would be beneficial if the durability of the electronic components is enhanced to improve the reliability of the electronic components and/or extend the effective cycle life of the surgical devices.

### SUMMARY

In one aspect of the present disclosure, a system includes a wiring harness and an encapsulation assembly for encapsulating a connection region between electronic components of the wiring harness. The wiring harness includes a flex cable and a force sensor electrically coupled to the flex cable at a connection region. The encapsulation assembly includes a mold having a first mold half and a second mold half. The first and second mold halves are configured to retain the force sensor therein and define a cavity above the connection region of the wiring harness.

In some aspects, the force sensor includes a proximal surface including a central region protruding outwardly from lateral regions extending from opposed sides of the central region, and the encapsulation assembly further includes a spacer having a band defining an opening therein. When the spacer is positioned on the force sensor, the band engages side walls of the central region of the force sensor. In certain aspects, the connection region of the wiring harness is between a pin block assembly disposed in one of the lateral regions of the force sensor and an electrical contact region of the flex cable, and the spacer includes a slit defined between end portions of the band. When the spacer is positioned on the force sensor, the slit is positioned against any of the side walls of the central region except for the side wall facing the pin block assembly.

The encapsulation assembly may further include a clamp. The encapsulation may further include an encapsulation material.

The first mold half may include a protrusion extending from an inner surface of the first mold half and, when the force sensor is positioned in the first mold, the protrusion extends through a central aperture of the force sensor. The first mold half may further include a recess defined therein, adjacent to the protrusion, that is configured to receive an electronic component extending from a distal surface of the force sensor.

The first mold half may include a channel defined around a portion of a periphery of an inner surface of the first mold half, and the second mold half may include a projection extending around a portion of a periphery of an inner surface of the second mold half. When the first and second mold halves are mated, the channel of the first mold half receives the projection of the second mold half therein.

The first mold half may include a wall extending from the inner surface. The wall may include an inner side facing the protrusion and an intermediate side extending at an angle and interconnecting the inner side with an outer side. When the force sensor is positioned in the first mold half, a first side of the force sensor engages the inner side of the wall. In some aspects, the first mold half includes a slit extending through the inner and outer surfaces of the first mold half. The slit is aligned with the intermediate side of the wall such that when the force sensor is positioned in the first mold half, a portion of the flex cable extends through the slit and against the intermediate side.

In some aspects, the second mold half includes an intermediate wall positioned and shaped to mate with the intermediate side of the first mold half to retain the flex cable therebetween. In some aspects, the second mold half includes a body defining an opening therein and, when the force sensor is disposed within the mated first and second mold halves, the proximal surface of the force sensor is accessible through the opening. In certain aspects, the second mold half includes an inner side and, when the force sensor is disposed within the mated first and second mold halves, a second side of the force sensor, opposed to the first side of the force sensor, engages the inner side of the second mold half.

In another aspect of the present disclosure, a method of encapsulating a connection region between electronic components includes: positioning a force sensor over a protrusion of a first mold half of a mold with a distal surface of the force sensor positioned against an inner surface of the first mold half and a first side of the force sensor abutting an inner side of a wall of the first mold half that extends outwardly from the inner surface; mounting a second mold half of the mold onto the first mold half to capture the force sensor within the mold, the second mold half including an inner side defining an opening therein such that the inner side of the second mold half abuts a second side of the force sensor and a proximal surface of the force sensor is accessible through the opening of the second mold half; and filling a cavity defined over a connection region formed between the force sensor and a flex cable with an encapsulation material through the opening defined in the second mold half.

In some aspects, the method further includes placing a spacer over a central region of the proximal surface of the force sensor prior to positioning the force sensor over the protrusion, the spacer including a band defining an opening therein that engages side walls of the central region of the force sensor.

The method may further include positioning a portion of the flex cable through a slit defined in the first mold half.

The method may further include securing the first and second mold halves together with a clamp.

In some aspects, the method further includes opening the mold after the encapsulation material has solidified within the cavity, and removing the force sensor from the mold.

Other aspects, features, and advantages will be apparent from the description, drawings, and the claims.

### BRIEF DESCRIPTION OF THE DRAWINGS

Various aspects of the present disclosure are described herein below with reference to the drawings, which are incorporated in and constitute a part of this specification, wherein:
FIG. 1 is a perspective view of a surgical device in accordance with an embodiment of the present disclosure;
FIG. 2 is a perspective view of an adapter assembly of the surgical device of FIG. 1;
FIG. 3 is a perspective view of a distal end portion of the adapter assembly of FIG. 2, with an outer sleeve of the adapter assembly removed therefrom;
FIGS. 4A-4D are perspective views of a force sensor of the adapter assembly of FIG. 3;
FIG. 5 is a perspective view of a wiring harness of the adapter assembly of FIG. 2;
FIG. 6 is a close-up view of a portion of the wiring harness of FIG. 5, including a spacer positioned over a central region of a force sensor in accordance with an embodiment of the present disclosure;
FIGS. 7 and 8 are perspective views of a mold, with first and second mold halves separated, that is configured to receive the force sensor and spacer of FIG. 6 therein in accordance with an embodiment of the present disclosure;
FIG. 9 is a perspective view of the force sensor of FIG. 6 positioned within the first mold half of the mold of FIGS. 7 and 8;
FIGS. 10 and 11 are perspective views of the force sensor of FIG. 6 positioned within the first and second mold halves of the mold of FIGS. 7 and 8;
FIGS. 12 and 13 are perspective views of the force sensor and mold of FIGS. 10 and 11 positioned within a clamp in accordance with an embodiment of the present disclosure;
FIG. 14 is a close-up view of the force sensor, the mold, and the clamp of FIG. 12, after an encapsulation material has been poured into a cavity defined in the mold;
FIG. 15 is a perspective view of the force sensor of FIG. 14, after the encapsulation material has solidified and the force sensor has been removed from the clamp and the mold; and
FIG. 16 is a plan view of the force sensor of FIG. 15.

### DETAILED DESCRIPTION

Surgical devices in accordance with embodiments of the present disclosure include electronic components hermetically sealed within an encapsulation material to protect the electronic components from exposure to moisture during, for example, cleaning and/or sterilizing procedures where the surgical devices may be subjected to high temperatures, steam, chemicals, and/or moisture. The electronic components of the surgical devices of the present disclosure are protected to prevent and/or resist breakdown over multiple/repeated cleaning and sterilizing cycles.

While the present disclosure is directed to protection of the connection region between a sensor and a flexible or flex cable of a surgical device, it is envisioned that the principles of the present disclosure are equally applicable to protection of a range of electronic components (e.g., printed circuit boards) and/or connection regions between various electronic components (e.g., solder connections) housed within reusable surgical devices.

Embodiments of the present disclosure are now described in detail with reference to the drawings in which like reference numerals designate identical or corresponding elements in each of the several views. Throughout this description, the term "proximal" refers to a portion of a structure, or component thereof, that is closer to a user, and the term "distal" refers to a portion of the structure, or component thereof, that is farther from the user. Directional reference terms, such as "top," "bottom," "side," and the like, are used to ease description of the embodiments and are not intended to have any limiting effect on the ultimate orientation of a structure or any part thereof.

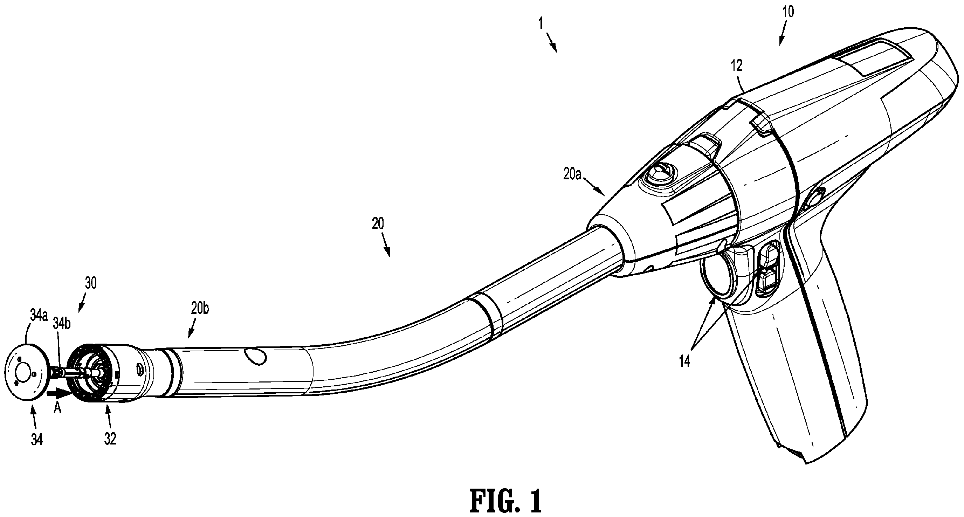

Turning now to FIG. 1, a surgical device 1 in accordance with an embodiment of the present disclosure is shown. The surgical device 1 is in the form of a powered handheld electromechanical surgical instrument, and includes a powered handle assembly 10, an adapter assembly 20, and a tool assembly or end effector 30. The powered handle assembly 10 is configured for selective connection with the adapter assembly 20 and, in turn, the adapter assembly 20 is configured for selective connection with the end effector 30.

The surgical device 1 will only further be described to the extent necessary to disclose aspects of the present disclosure. For a detailed description of the structure and function of exemplary surgical devices (e.g., handle assemblies, adapter assemblies, and/or end effectors), reference may be made to commonly owned U.S. Patent Publication Nos. 2016/0296234, 2016/0310134, and 2018/0360460, the entire content of each of which is incorporated herein by reference.

With continued reference to FIG. 1, the handle assembly 10 includes a handle housing 12 housing a power-pack (not shown) configured to power and control various operations of the surgical device 1, and a plurality of actuators 14 (e.g., finger-actuated control buttons, knobs, toggles, slides, interfaces, and the like) for activating various functions of the surgical device 1. The adapter assembly 20 includes a proximal portion 20a configured for operable connection to the handle assembly 10 and a distal portion 20b configured for operable connection to the end effector 30. The end effector 30 includes a loading unit 32 having a plurality of staples (not shown) disposed therein and an anvil assembly 34 including an anvil head 34a and an anvil rod 34b.

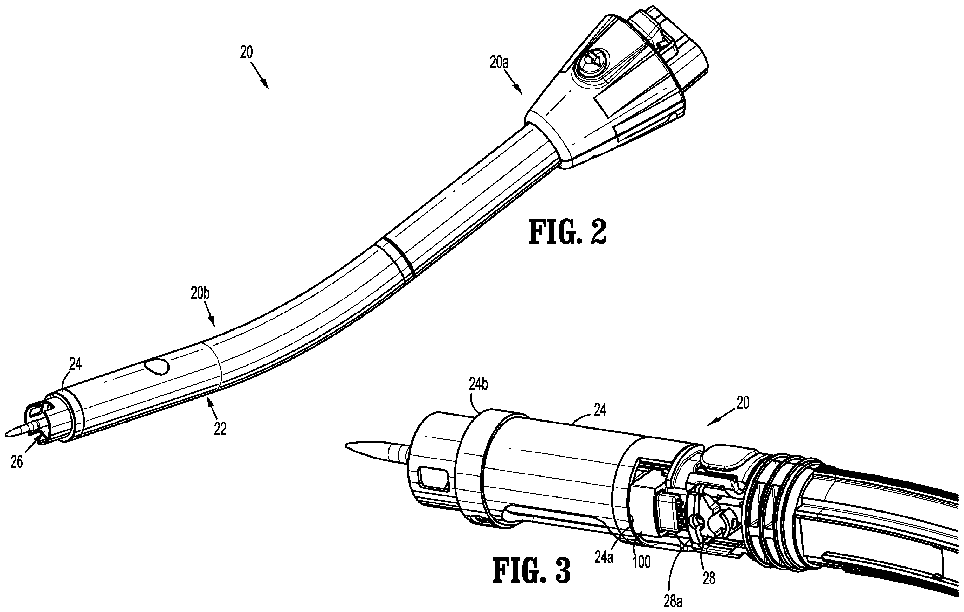

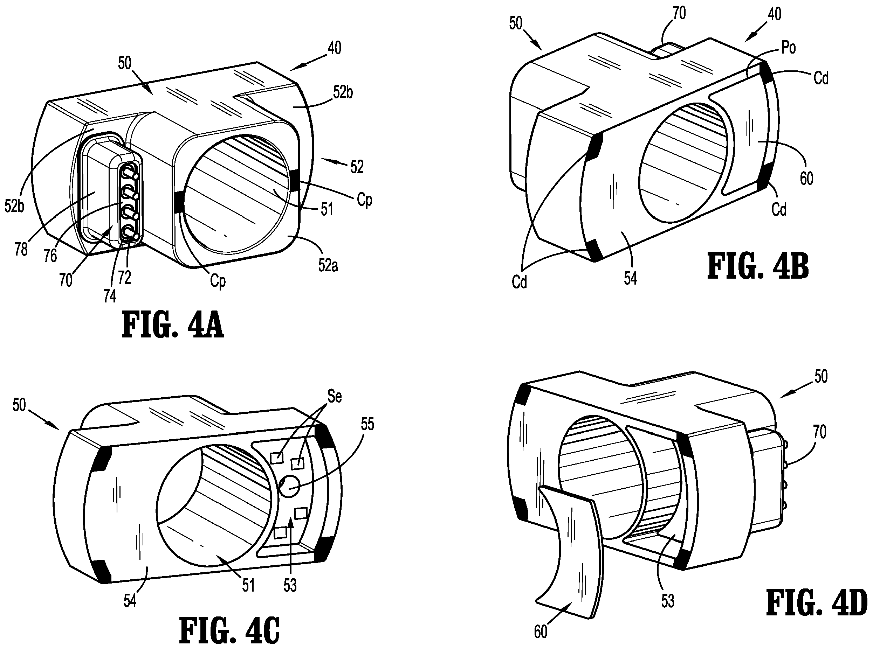

Referring now to FIGS. 2 and 3, in conjunction with FIG. 1, the adapter assembly 20 includes an outer tube or sleeve 22, and a connector housing 24 secured to a distal end of the outer sleeve 22 that is configured to releasably secure an end effector, e.g., the end effector 30 (FIG. 1), to the adapter assembly 20. The adapter assembly 20 further includes a trocar assembly 26 that extends through a central aperture 51 (FIG. 4A) of a force sensor 40 and a central aperture (not shown) of a trocar connection housing 28. The trocar connection housing 28 releasably secures the trocar assembly 26 relative to the outer sleeve 22 of the adapter assembly 20.

The force sensor 40 is disposed between the trocar connection housing 28 and the connector housing 24 of the adapter assembly 20, and is configured to measure forces along a load path. The trocar connection housing 28 includes a distal surface 28a which interfaces with, and loads a proximal surface 52 (FIG. 4A) of a body or substrate 50 of the force sensor 40 at proximal load contact areas "Cp," and a proximal surface 24a of the connector housing 24 defines a contact surface which loads a distal surface 54 (FIG. 4B) of the substrate 50 of the force sensor 40 at distal load contact areas "Cd." Thus, for example, as the anvil assembly 34 (FIG. 1) is approximated towards the loading unit 32 of the end effector 30 during clamping and/or stapling of tissue, the anvil head 34a applies uniform pressure in the direction of arrow "A" (FIG. 1) against a distal surface 24b of the connector housing 24 which, in turn, is transmitted to the distal load contact areas "Cd" of the force sensor 40.

The force sensor 40 will only further be described to the extent necessary to disclose aspects of the present disclosure. For a detailed description of the structure and function of exemplary sensors (e.g., force sensors), reference may be made to commonly owned U.S. Patent Publication Nos. 2018/0042689, 2018/0042610, and 2018/0067004, the entire content of each of which is incorporated herein by reference.

Referring now to FIGS. 4A-4D, the force sensor 40 includes a substrate 50, a distal or first plate 60, and a pin block assembly 70. The substrate 50 includes a central aperture 51 defined therethrough and extending between proximal and distal surfaces 52, 54 of the substrate 50. The proximal and distal surfaces 52, 54 are load bearing surfaces (the proximal surface 52 interfacing with the trocar connection housing 28, and the distal surface 54 interfacing with the connector housing 24, as described above) that allow the substrate 50 to flex when loaded by a surgical device.

The proximal surface 52 of the substrate 50 is a stepped surface including a central region 52a, which is an outwardly protruding loading portion, and lateral regions 54b extending from opposed sides of the central region 52a. The distal surface 54 of the substrate 50 is a substantially planar surface and defines a distal or first recess 53 therein on one side of the central aperture 51 of the substrate 50. A through-hole 55 extends through the distal recess 53 and one of the lateral regions 54b of the proximal surface 52 of the substrate 50. The through-hole 55 is aligned with the pin block assembly 70 which is secured to the lateral region 54b of the proximal surface 52 of the substrate 50.

Sensing elements "Se," e.g., strain gauges, are disposed (e.g., secured or bonded) within the distal recess 53, along with associated components thereof (not shown), e.g., media layers, films, protective coatings, circuitry including electronic components, such as resistors, and conductive wires and/or traces, electronic and/or solder connectors, etc. The sensing elements "Se" are mounted in specific locations within the distal recess 53 and are connected together with a series of wires (not shown) to form a resistance bridge, e.g., a Wheatstone bridge, that can read a linear strain response of the substrate 50 when compressed, as is within the purview of those skilled in the art.

The distal plate 60 covers the distal recess 53 and is secured (e.g., welded) to the distal surface 54 of the substrate 50 around the entire outer perimeter "Po" of the distal plate 60 to form a hermetic seal and leak-proof barrier protecting the sensing elements "Se" and associated components from the external environment (e.g., during cleaning and/or sterilization processes). Wires (not shown) that are soldered to the sensing elements "Se," are passed through the through-hole 55 and secured (e.g., soldered) to conductive pins 72 of the pin block assembly 70 so that electrical signals may exit the substrate 50 in order to supply power and read force responses from the force sensor 40, while allowing the internal wires and electronics to be protected from the outside environment.

The pin block assembly 70 includes a plurality of conductive pins 72, with each pin 72 extending through a glass substrate or seal 74 disposed within an opening of a pin block housing 76 which is housed within a pin block cover 78. The pin block assembly 70 is secured in a fluid tight manner to the lateral region 52b of the proximal surface 52 of the substrate 50 such that the through-hole 55 provides a hermetically sealed pathway between the sensing elements "Se" and distal portions of the plurality of conductive pins 72 that are sealed within the pin block assembly 70.

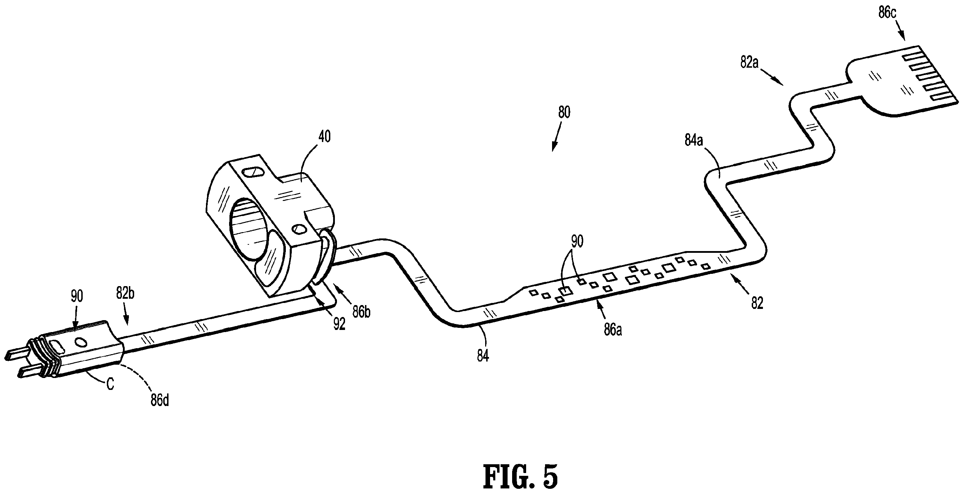

With reference now to FIG. 5, in conjunction with FIG. 1, force sensor 40 is electrically coupled to a wiring harness 80 of the adapter assembly 20 that electronically interconnects the handle assembly 10 and the end effector 30 of the surgical device 1. The wiring harness 80 is configured to enable communication between the handle assembly 10 and the end effector 30, and to relay power from the handle assembly 10 to the end effector 30. For example, this communication allows for calibration and communication of data and control signals between the end effector 30 and the adapter assembly 20, as well as between the adapter assembly 20 and the handle assembly 10, thereby transferring data pertaining to the end effector 30 to the handle assembly 10 and signals from the handle assembly 10 to the end effector 30.

The wiring harness 80 includes a flex cable 82 including a body or substrate 84 suitable for supporting and/or electrically connecting electronic components 90 thereto. The substrate 84 is formed from one or more layers or sheets of dielectric material, such as a polymer or a ceramic, and one or more layers of conductive material, such as copper foil, that form conductive traces (not explicitly shown) in the substrate 84. Vias (not shown) may interconnect the conductive traces through different layers of the flex cable 82. It should be understood that the substrate 84 is configured to allow for the fabrication of single or double sided flex circuits, multilayer flex circuits, and rigid flex circuits.

A plurality of electrical contact regions 86 (referred to herein as first, second, third, and fourth electrical contact regions 86a-d) are disposed at terminal ends of the conductive traces (not shown) defined through the substrate 84 on a first side 84a of the flex cable 82. It should be understood that while the flex cable 82 is shown including four electrical contact regions 86, the flex cable 82 may have any number of electrical contact regions depending upon the desired configuration and functionality of the wiring harness 80, as is within the purview of those skilled in the art.

Each of the plurality of electrical contact regions 86 includes one or more pads (e.g., solder pads) to which electronic components 90 are joined (e.g., soldered). The electronic components 90 may be, for example, surface mount technology and/or through-hole technology, including, for example, integrated circuits (e.g., microchips, microcontrollers, microprocessors), resistors, amplifiers, inductors, capacitors, sensing elements (e.g., optical sensors, pressure sensors, capacitive sensors), buttons, switches, circuit boards, electrical connectors, cables, and/or wires, among other elements or circuitry within the purview of those skilled in the art.

The first electrical contact region 86a includes electronic components 90 disposed thereon that form of an electronic circuit. The second electrical contact region 86b is aligned and soldered to the force sensor 40 for measuring forces of the end effector 30. A proximal portion 82a of the substrate 82 includes the third electrical contact region 86c configured for electrical connection with the handle assembly 10 (FIG. 1). A distal portion 82b of the substrate 82 includes the fourth electrical contact region 86d (shown in phantom) coupled to an electronic component 90 in the form of an electrical connector "C" for electrical connection with the end effector 30 (FIG. 1).

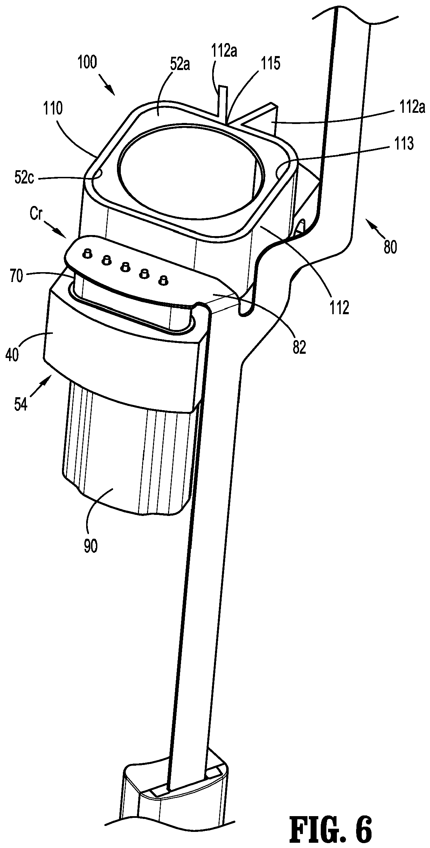

Referring now to FIGS. 6-8, an encapsulation assembly 100 for encapsulating and hermetically sealing the connection region "Cr" (e.g., solder connection) between the pin block assembly 70 of the force sensor 40 and the flex cable 82 of the wiring harness 80 is shown. As initially seen in FIG. 6, the encapsulation assembly 100 includes a spacer or sleeve 110. The spacer 110 includes a band 112 defining an opening 113 therethrough. The band 112 is configured to slip onto and be secured to the central region 52a of the force sensor 40 by, for example, a friction or pressure fit. The band 112 is sized and shaped to fit around, engage (e.g., grip), and cover side walls 52c of the central region 52a of the force sensor 40 such that the central region 52a of the force sensor 40 is received within the opening 113 of the spacer 110.

The spacer 110 may be fabricated from a resilient material, such as, for example, silicone or rubber, and/or a lubricious material such as, for example, a synthetic polymer (e.g., polyoxymethylene, acetal homopolymer, or polytetrafluoroethylene). In embodiments, the spacer 110 may be coated with a release agent, or a lubricious material to reduce the need for a release agent.

The band 112 of the spacer 110 is shaped such that the opening 113 defined therein is discontinuous and a slit 115 is defined between end portions 112a of the band 112. The end portions 112a extend outwardly from the band 112 and may be utilized by a user for placement and/or removal of the spacer 110 from the force sensor 40. The slit 115 may be positioned against any of the side walls 52c of the force sensor 40 except for the side wall 52c facing the pin block assembly 70. The side wall 52c facing the pin block assembly 70 is covered by a continuous expanse of the band 112. Alternatively, the band 112 may be shaped such that the opening 113 defined therein is closed within the spacer 110 such that the band 112 frictionally engages the entirety of the side walls 52c of the central region 52a of the force sensor 40.

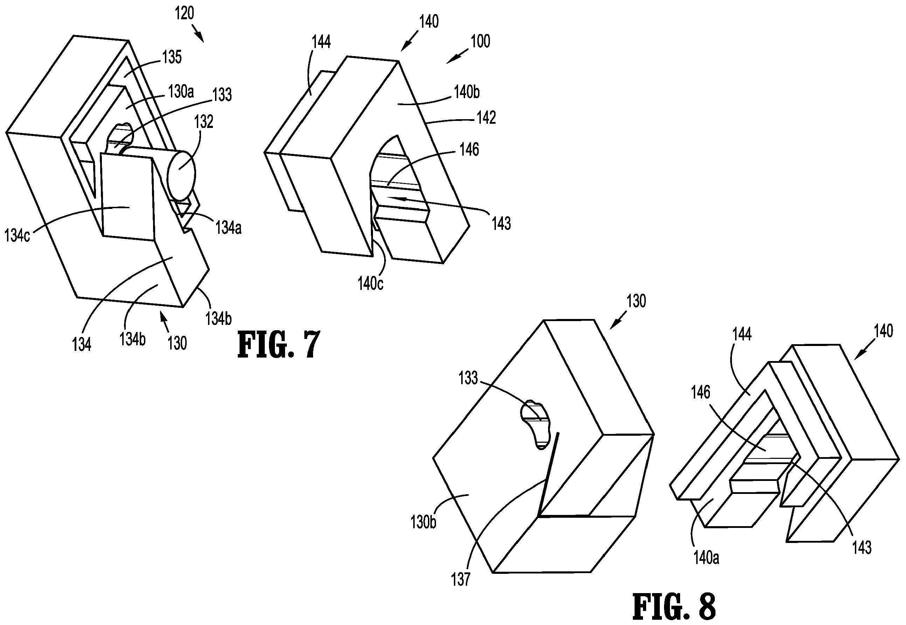

As seen in FIGS. 7 and 8, the encapsulation assembly 100 further includes a mold 120 including first and second mold halves or portions 130, 140. The first mold half 130 includes a first or inner surface 130a configured to receive the force sensor 40 (FIG. 4A) and mate with the second mold half 140, and a second or outer surface 130b. The inner surface 130a includes a protrusion 132 extending from about the center of the inner surface 130a. The protrusion 132 is sized and shaped to be received within the central aperture 51 (FIG. 4A) of the force sensor 40 such that the distal surface 54 (FIG. 4B) of the substrate 50 of the force sensor 40 is positioned against the inner surface 130a of the first mold half 130. As shown, the protrusion 132 is a cylindrical peg sized and shaped to engage and retain the force sensor 40 to the first mold half 130, however, the protrusion 132 may be any shape having a complementary geometry to the central aperture 51 of the force sensor 40. Adjacent to the protrusion 132 is a recess or opening 133 sized and shaped to receive an electronic component 90 (FIG. 6) of the wiring harness 80 extending from the distal surface 54 of the force sensor 40.

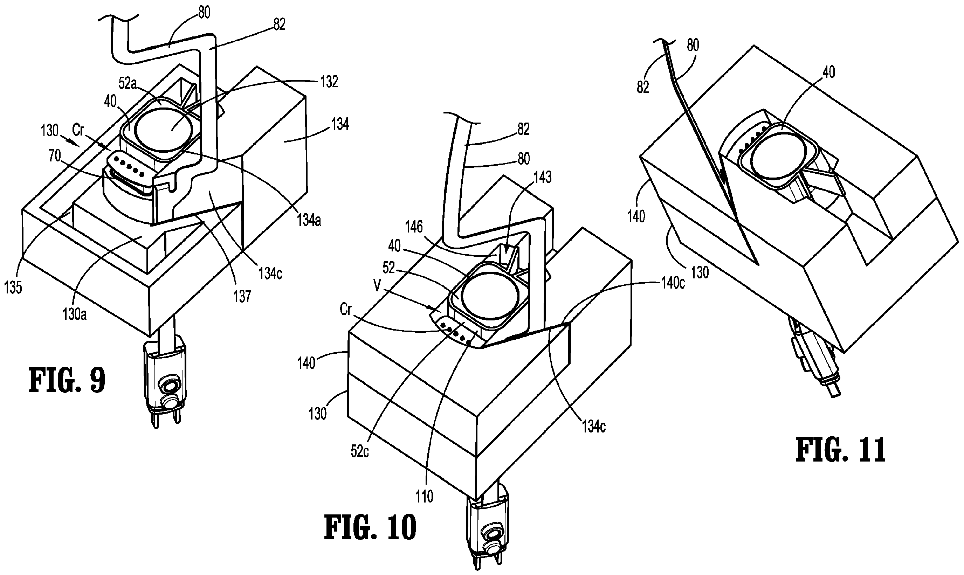

A channel or groove 135 is defined around a portion of the periphery of the inner surface 130a of the first mold half 130 and is configured to receive a mating projection or tongue 144 of the second mold half 140 during assembly. The channel 135 has a substantially L-shaped or U-shaped configuration, however, it should be understood that the channel 135 may have any shape complementary to the shape of the projection 144. A wall 134 extends outwardly from the inner surface 130a of the first mold half 130. The wall 134 includes an inner or first side 134a facing the protrusion 132, outer or second sides 134b aligned with outer side surfaces 130c of the first mold half 130, and a third or intermediate side 134c extending at an angle and interconnecting the inner and outer sides 134a, 134b. A slit 137 extends through the inner and outer surfaces 130a, 130b of the first mold half 130, and is aligned with the intermediate side 134c of the wall 134 (see e.g., FIG. 9).

The wall 134 and channel 135 of the first mold half 130 are positioned relative to each other such that when the force sensor 40, with spacer 110 attached, is disposed within the first mold half 130, as shown in FIG. 9, the inner side 134a of the wall 134 of the first mold half 130 engages (e.g., abuts) one side of the force sensor 40 and, when the second mold half 140 is mated to the first mold half 130, as shown in FIG. 10, an inner side 146 of the second mold half 140 that defines the opening 143 thereof engages another side of the force sensor 40 opposed to the side the inner surface 134a of the wall 134 of the first mold half 130 is positioned against to stabilize the force sensor 40 therebetween. The position of the slit 137 retains a portion of the flex cable 82 thereagainst without deforming (e.g., twisting, bending, etc.) and compromising the integrity of the flex cable 82 upon placement of the force sensor 40 within the first mold half 130.

With continued reference to FIGS. 7 and 8, the second mold half 140 has a substantially L-shaped or U-shaped body 142 defining an opening 143 about the center of the body 142. The opening 143 is configured to let the force sensor 40 (FIG. 4A) sit therein with the proximal surface 52 of the force sensor 40 accessible therethrough. The second mold half 140 includes a first or inner surface 140a configured to mate with the inner surface 130a of the first mold half 130, and a second or outer surface 140b. The inner surface 140a of the second half 140 includes a projection 144 extending therefrom that is configured to mate with the channel 135 defined in the first mold half 130, as discussed above, such that when the first and second mold halves 130, 140 are joined together, the outer surfaces 130b, 140b align and are coterminous with each other, and the force sensor 40 is secured within the mold 120. The second mold half 140 further includes an intermediate surface 140c positioned and shaped to mate with the intermediate side 134c of the first mold half 130 to retain the flex cable 82 (FIG. 5) therebetween.

As shown in FIGS. 9-14, in a method of encapsulating the connection region "Cr" between the pin block assembly 70 of the force sensor 40 and the flex cable 82, the spacer 110 is positioned around the central region 52a of the force sensor 40, as described and shown above with respect to FIG. 6. As seen in FIG. 9, the force sensor 40, with attached spacer 110, is positioned in the first mold half 130 by threading a portion of the first flex cable 82 through the slit 137 defined in the first mold half 130 and sliding the force sensor 40 over the protrusion 132 of the first mold half 130 such that the distal surface 54 (FIG. 4B) of the force sensor 40 abuts the inner surface 130a of the first mold half 130, and a side of the force sensor 40 abuts the inner surface 134a of the wall 134 of the first mold half 130. The inner surface 134a of the wall 134 is contoured to match the dimensions of the side of the force sensor 40 against which it is positioned for tight engagement therebetween.

As seen in FIGS. 10 and 11, in conjunction with FIG. 9, the second mold half 140 is then placed over the first mold half 130 such that the projection 144 (FIG. 8) of the second mold half 140 engages the channel 135 of the first mold half 130 and the flex cable 82 is secured between the intermediate side 134c of the first mold half 130 and the intermediate surface 140c of the second mold half 140. In this assembled state, the proximal surface 52 of the force sensor 40 is exposed through the opening 143 of the second mold half 140, and a cavity "V" is disposed over the connection region "Cr" between the force sensor 40 and the flex cable 82. The cavity "V" is defined by portions of the first and second mold halves 130, 140, and the spacer 110 covering the side wall 52c of the substrate 50 facing the pin block assembly 70. The outer surface 140b of the second mold half 140 is disposed at a height that matches the height of the protrusion 132 of the first mold half 130 such that the height of the cavity "V" coincides with the height of the central region 52a of the force sensor 40.

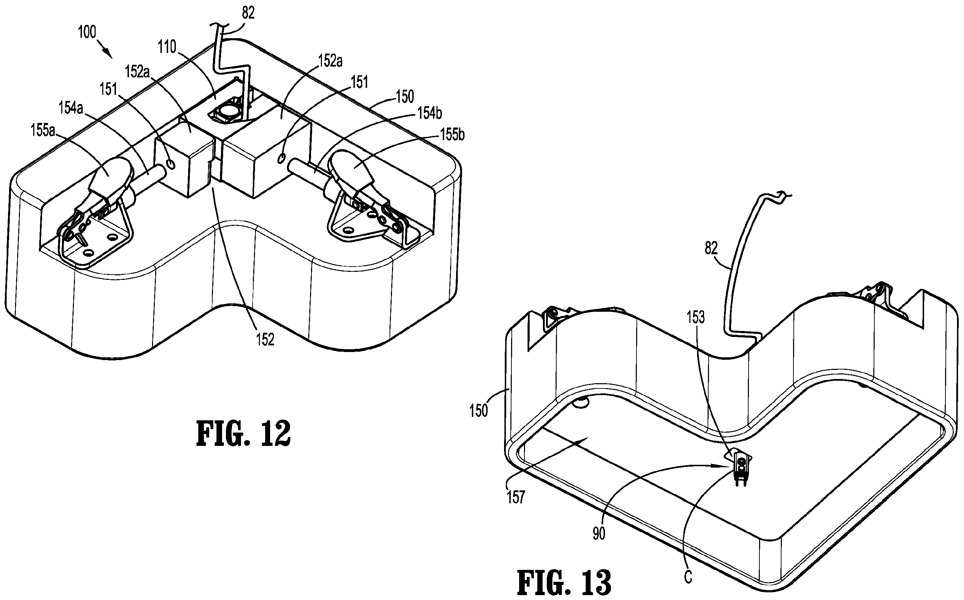

After the first and second mold halves 130, 140 are mated, the first and second mold halves 130, 140 may be releasably secured together by various mechanical and/or motorized devices such as, for example, clamps, bands, tape, etc. As shown in FIGS. 12 and 13, the encapsulation assembly 100 may further include a clamp 150 into which the assembled mold 110 is placed. The clamp 150 is a dual action clamp including a clamp bed 152 in which the assembled mold 110 is placed, and first and second clamps 154a, 154b (e.g., linear toggle clamps) configured to interface with adjacent sides of the mold 110 through openings 151 defined through clamp pads 152a of the clamp bed 152. The first and second clamps 154a, 154b are threadably couplable to the respective clamp pads 152a to allow for adjustment in clamping pressure when a toggle 155a, 155b of the respective first and second clamps 154a, 154b is locked to maintain consistent clamping force. The clamp 150 includes an aperture 153 extending through the clamp bed 152 to an undersurface of the clamp 150 for passage of the flex cable 82 therethrough, and the underside of the clamp 150 defines a flanged cavity 157 to provide space for the flex cable 82 as well as any electronic components 90 coupled thereto (e.g., the electrical connector "C" coupled to the flex cable 82).

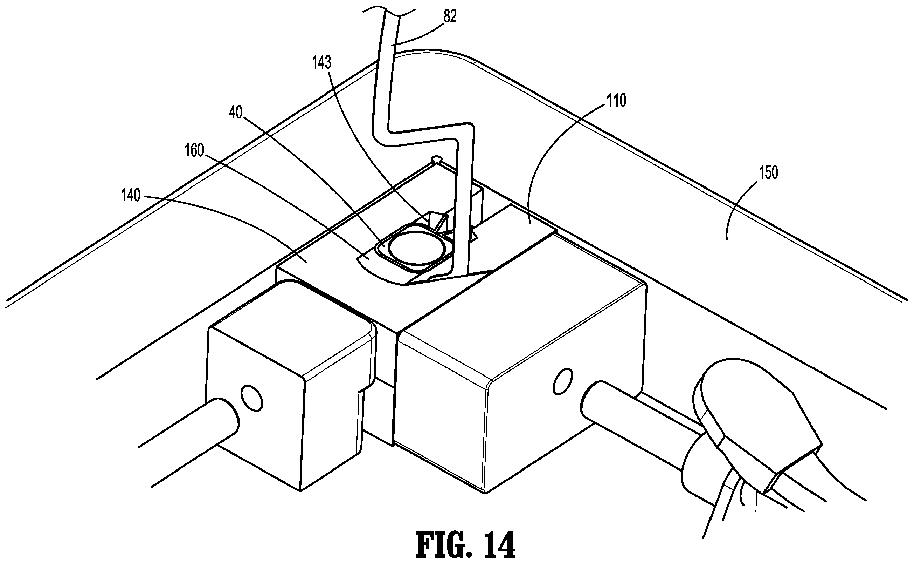

As seen in FIG. 14, an encapsulation material 160 is then injected into the cavity "V" (FIG. 10) defined in the mold 110 above the connection region "Cr", through the opening 143 of the second mold half 140 until the encapsulation material 160 fills the cavity "V." The encapsulation material 160 may be, for example, resins, urethanes, acrylics, epoxies, among other materials that are flexible in nature so that the encapsulation material can move with the thermal and mechanical movement of the force sensor 40 and the flex cable 82, and withstanding cleaning and sterilization cycles. The encapsulation material 160 is rendered into a liquid state for injection or pouring into the mold 110 by any method suitable for the type of encapsulation material utilized, as is within the purview of those skilled in the art. For example, the encapsulation material may be mixed, blended, and/or heated to activate or make the encapsulation material flowable.

Casting of the encapsulation material 160 may be performed using vacuum or in the presence of an inert gas (e.g., argon, nitrogen, etc.), as is within the purview of those skilled in the art. In embodiments, prior to injecting or pouring the encapsulation material 160 into the mold 110, a vacuum is applied to the encapsulation material 160. In some embodiments, the encapsulation material 160 is mixed and/or heated, poured into the mold 110, and then placed into a vacuum chamber. By pulling vacuum when the encapsulation material 160 is in the liquid state, any bubbles, voids, and fluid porosity can be evacuated to form a solid encapsulate. In certain embodiments, the encapsulation material 160, the mold 110, and the clamp 150 are placed in a vacuum or inert gas environment, and the encapsulation material 160 is mixed, casted, and cured under vacuum.

The encapsulation material 160 remains in the mold 110 until it has solidified or cured. Thereafter, the mold 110 is removed from the clamp 150 by unclamping the first and second mold halves 130, 140, and the force sensor 40 is de-molded by removing the first and second mold halves 130, 140, and the spacer 110. The first and second mold halves 130, 140 may be formed from or coated with a lubricious material, or a release agent may be applied to first and second mold halves 130, 140 for ease of removal of the force sensor 40 therefrom.

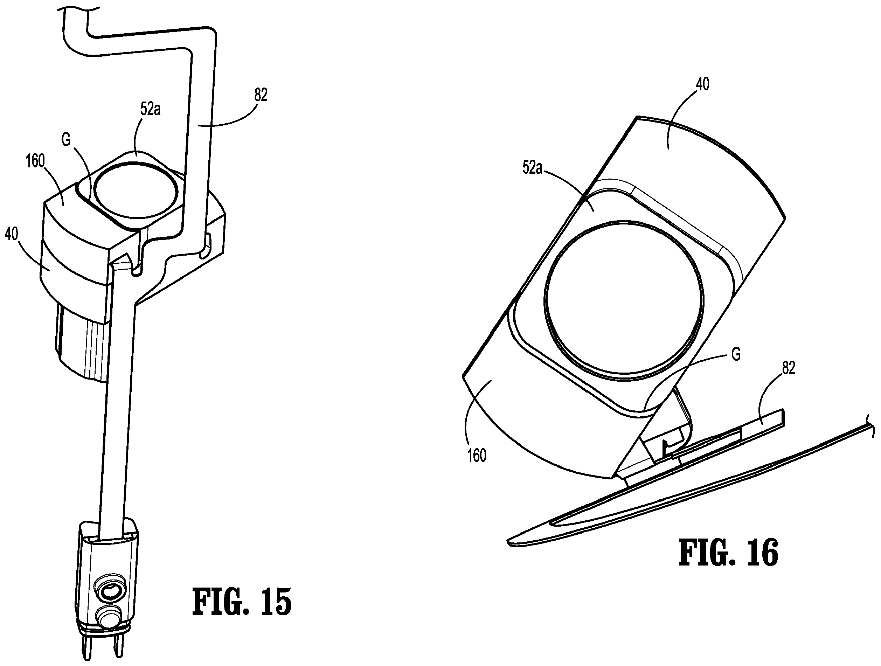

As shown in FIGS. 15 and 16, the force sensor 40 includes a substantially co-radial and co-planar encapsulation material 160 around the entirety of the connection region "Cr" (FIG. 6) between the force sensor 40 and the flex cable 82. A gap "G" is defined between the encapsulation material 160 and the central region 52a of the force sensor 40, due to use of the spacer 110 during encapsulation, so that the encapsulation material 160 does not interfere with sensor bending.

It should be understood that the sensor shown and described above may have other configurations, and the spacer and/or the mold may be modified and configured to receive the sensors therein to provide a receiving zone for the encapsulation material over various connection regions of the sensor to electronic components. Similarly, the flex cable and/or wiring hardness may have different configurations, and the mold and/or clamp may be modified to accommodate and receive portions of the flex cable and/or wiring harness therebetween and/or therethrough.

Persons skilled in the art will understand that the structures specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. For example, the assemblies and methods of the present disclosure may be utilized with electronic components disposed in other components of the surgical devices, or in other surgical devices, such as robotic or powered surgical devices/instruments that are subject to washing and/or sterilization procedures. Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:-
1. A system including a wiring harness and an encapsulation assembly for encapsulating a connection region between electronic components of the wiring harness, the system comprising:
   a wiring harness including:
      a flex cable; and
      a force sensor electrically coupled to the flex cable at a connection region; and
   an encapsulation assembly including:
      a mold including a first mold half and a second mold half, the first and second mold halves configured to retain the force sensor therein and define a cavity above the connection region of the wiring harness.
2. The system according to paragraph 1, wherein the force sensor includes a proximal surface including a central region protruding outwardly from lateral regions extending from opposed sides of the central region, and wherein the encapsulation assembly further includes a spacer having a band defining an opening therein and, when the spacer is positioned on the force sensor, the band engages side walls of the central region of the force sensor.
3. The system according to paragraph 2, wherein the connection region of the wiring harness is between a pin block assembly disposed in one of the lateral regions of the force sensor and an electrical contact region of the flex cable, and wherein the spacer includes a slit defined between end portions of the band and, when the spacer is positioned on the force sensor, the slit is positioned against any of the side walls of the central region except for the side wall facing the pin block assembly.
4. The system according to paragraph 1, wherein the encapsulation assembly further includes a clamp.
5. The system according to paragraph 1, wherein the encapsulation assembly further includes an encapsulation material.
6. The system according to paragraph 1, wherein the first mold half includes a protrusion extending from an inner surface of the first mold half and, when the force sensor is positioned in the first mold, the protrusion extends through a central aperture of the force sensor.
7. The system according to paragraph 6, wherein the first mold half further includes a recess defined therein, adjacent to the protrusion, the recess configured to receive an electronic component extending from a distal surface of the force sensor.
8. The system according to paragraph 1, wherein the first mold half includes a channel defined around a portion of a periphery of an inner surface of the first mold half, and the second mold half includes a projection extending around a portion of a periphery of an inner surface of the second mold half, and when the first and second mold halves are mated, the channel of the first mold half receives the projection of the second mold half therein.
9. The system according to paragraph 6, wherein the first mold half includes a wall extending from the inner surface, the wall including an inner side facing the protrusion and an intermediate side extending at an angle and interconnecting the inner side with an outer side and, when the force sensor is positioned in the first mold half, a first side of the force sensor engages the inner side of the wall.
10. The system according to paragraph 9, wherein the first mold half includes a slit extending through the inner and outer surfaces of the first mold half, the slit aligned with the intermediate side of the wall such that when the force sensor is positioned in the first mold half, a portion of the flex cable extends through the slit and against the intermediate side.
11. The system according to paragraph 9, wherein the second mold half includes an intermediate wall positioned and shaped to mate with the intermediate side of the first mold half to retain the flex cable therebetween.
12. The system according to paragraph 9, wherein the second mold half includes a body defining an opening therein and, when the force sensor is disposed within the mated first and second mold halves, the proximal surface of the force sensor is accessible through the opening.
13. The system according to paragraph 12, wherein the second mold half includes an inner side and, when the force sensor is disposed within the mated first and second mold halves, a second side of the force sensor, opposed to the first side of the force sensor, engages the inner side of the second mold half.
14. A method of encapsulating a connection region between electronic components, the method comprising:
   positioning a force sensor over a protrusion of a first mold half of a mold with a distal surface of the force sensor positioned against an inner surface of the first mold half and a first side of the force sensor abutting an inner side of a wall of the first mold half that extends outwardly from the inner surface;
   mounting a second mold half of the mold onto the first mold half to capture the force sensor within the mold, the second mold half including an inner side defining an opening therein such that the inner side of the second mold half abuts a second side of the force sensor and a proximal surface of the force sensor is accessible through the opening of the second mold half; and
   filling a cavity defined over a connection region formed between the force sensor and a flex cable with an encapsulation material through the opening defined in the second mold half.
15. The method according to paragraph 14, further comprising placing a spacer over a central region of the proximal surface of the force sensor prior to positioning the force sensor over the protrusion, the spacer including a band defining an opening therein that engages side walls of the central region of the force sensor.
16. The method according to paragraph 14, further comprising positioning a portion of the flex cable through a slit defined in the first mold half.
17. The method according to paragraph 14, further comprising securing the first and second mold halves together with a clamp.
18. The method according to paragraph 14, further comprising:
   opening the mold after the encapsulation material has solidified within the cavity; and
   removing the force sensor from the mold.

## Claims

1. A system including a wiring harness and an encapsulation assembly for encapsulating a connection region between electronic components of the wiring harness, the system comprising:
a wiring harness including:
a flex cable; and
a force sensor electrically coupled to the flex cable at a connection region; and
an encapsulation assembly including:
a mold including a first mold half and a second mold half, the first and second mold halves configured to retain the force sensor therein and define a cavity above the connection region of the wiring harness.

2. The system according to claim 1, wherein the force sensor includes a proximal surface including a central region protruding outwardly from lateral regions extending from opposed sides of the central region, and wherein the encapsulation assembly further includes a spacer having a band defining an opening therein and, when the spacer is positioned on the force sensor, the band engages side walls of the central region of the force sensor.

3. The system according to claim 2, wherein the connection region of the wiring harness is between a pin block assembly disposed in one of the lateral regions of the force sensor and an electrical contact region of the flex cable, and wherein the spacer includes a slit defined between end portions of the band and, when the spacer is positioned on the force sensor, the slit is positioned against any of the side walls of the central region except for the side wall facing the pin block assembly.

4. The system according to any preceding claim, wherein the encapsulation assembly further includes a clamp.

5. The system according to any preceding claim, wherein the encapsulation assembly further includes an encapsulation material.

6. The system according to any preceding claim, wherein the first mold half includes a protrusion extending from an inner surface of the first mold half and, when the force sensor is positioned in the first mold, the protrusion extends through a central aperture of the force sensor; preferably wherein the first mold half further includes a recess defined therein, adjacent to the protrusion, the recess configured to receive an electronic component extending from a distal surface of the force sensor.

7. The system according to any preceding claim, wherein the first mold half includes a channel defined around a portion of a periphery of an inner surface of the first mold half, and the second mold half includes a projection extending around a portion of a periphery of an inner surface of the second mold half, and when the first and second mold halves are mated, the channel of the first mold half receives the projection of the second mold half therein; and/or wherein the first mold half includes a wall extending from the inner surface, the wall including an inner side facing the protrusion and an intermediate side extending at an angle and interconnecting the inner side with an outer side and, when the force sensor is positioned in the first mold half, a first side of the force sensor engages the inner side of the wall.

8. The system according to claim 7, wherein the first mold half includes a slit extending through the inner and outer surfaces of the first mold half, the slit aligned with the intermediate side of the wall such that when the force sensor is positioned in the first mold half, a portion of the flex cable extends through the slit and against the intermediate side; and/or wherein the second mold half includes an intermediate wall positioned and shaped to mate with the intermediate side of the first mold half to retain the flex cable therebetween.

9. The system according to claim 8, wherein the second mold half includes a body defining an opening therein and, when the force sensor is disposed within the mated first and second mold halves, the proximal surface of the force sensor is accessible through the opening.

10. The system according to claim 9, wherein the second mold half includes an inner side and, when the force sensor is disposed within the mated first and second mold halves, a second side of the force sensor, opposed to the first side of the force sensor, engages the inner side of the second mold half.

11. A method of encapsulating a connection region between electronic components, the method comprising:
positioning a force sensor over a protrusion of a first mold half of a mold with a distal surface of the force sensor positioned against an inner surface of the first mold half and a first side of the force sensor abutting an inner side of a wall of the first mold half that extends outwardly from the inner surface;
mounting a second mold half of the mold onto the first mold half to capture the force sensor within the mold, the second mold half including an inner side defining an opening therein such that the inner side of the second mold half abuts a second side of the force sensor and a proximal surface of the force sensor is accessible through the opening of the second mold half; and
filling a cavity defined over a connection region formed between the force sensor and a flex cable with an encapsulation material through the opening defined in the second mold half.

12. The method according to claim 11, further comprising placing a spacer over a central region of the proximal surface of the force sensor prior to positioning the force sensor over the protrusion, the spacer including a band defining an opening therein that engages side walls of the central region of the force sensor.

13. The method according to claim 11 or claim 12, further comprising positioning a portion of the flex cable through a slit defined in the first mold half.

14. The method according to any of claims 11 to 13, further comprising securing the first and second mold halves together with a clamp.

15. The method according to any of claims 11 to 14, further comprising:
opening the mold after the encapsulation material has solidified within the cavity; and removing the force sensor from the mold.
